# EUROPÄISCHE PATENTANMELDUNG

(11) **EP 1 444 953 A1**
(43) Veröffentlichungstag der Anmeldung: **11.08.2004**
(21) Anmeldenummer: 03405070.8
(22) Anmeldetag: 10.02.2003
(51) Int. Cl.: A61B 8/12

(54) **Ultraschallsonde für ein stereoskopisches Abbildungssystem**

(71) Anmelder: Sulzer Markets and Technology AG, 8401 Winterthur (CH)
(72) Erfinder: Hirt, Felix, Dr., 8492 Wila (CH); Moser, Urs, Dr., 8006 Zürich (CH)
(74) Vertreter: Sulzer Management AG

(57) **Zusammenfassung**

Die Ultraschallsonde (1) ist für ein stereoskopisches Abbildungssystem vorgesehen, das auf einer rasterartigen Aufnahme von Daten und deren elektronischen Verarbeitung beruht. Die Sonde umfasst Gruppen (12) von Schallwandlern (11), die innerhalb einer elastomeren Hülle (10) auf einem zylinderischen, um eine Longitudinalachse rotierbaren Träger angeordnet sind. Der Träger ist aus mindestens zwei Segmenten (3, 4) zusammengesetzt. Die Segmente sind um einen Kippwinkel gegeneinander kippbar, und zwar um eine Kippachse, die transversal zur Longitudinalachse und vorzugsweise exzentrisch zu dieser liegt. Es gibt einen stabilen Lagezustand, in dem die Segmente unter Einwirkung einer dynamischen und/oder statischen Rückstellkraft eine Lage mit Ausrichtung in Richtung der Longitudinalachse einnehmen.

## Beschreibung

Die Erfindung betrifft eine Ultraschallsonde für ein stereoskopisches Abbildungssystem, das dazu verwendbar ist, an einem Patienten aus einem Körperinnenraum heraus dreidimensionale Darstellungen eines Organs herzustellen. Die Erfindung bezieht sich auch auf die Verwendung dieser Sonde.

Aus der EP-A- 0 926 491 ist eine Ultraschall-Messvorrichtung für ein bildgebendes Verfahren bekannt. Eine Sonde dieser Vorrichtung ist ein stabförmiger Körper, der eine Mehrzahl von Wandlerelementen trägt. Die Wandlerelemente sind Transducer, die Ultraschallsignale aussenden und reflekierte Komponenten dieser Signale empfangen. Von einer Transducerfläche aus wird senkrecht zur Fläche eine Schallkeule abgestrahlt. In einer bevorzugten Ausführungsform der Sonde ist der stabförmige Träger um eine Longitudinalachse drehbar.

Die Sonde ist für Behandlungsverfahren entwickelt worden, bei denen ein Katheter in das ungeöffnete, schlagende Herz eines Patienten eingeführt und dort positioniert wird. Mit der Sonde, die in die Speiseröhre eingebracht wird, werden die für eine Navigation des Katheters benötigten Daten gewonnen. Aus den Daten muss mit einem Echtzeitverfahren ein stereoskopisches Bild berechnet werden, das eine ausreichende Auflösung hat, so dass der Operateur zu jedem Zeitpunkt in der Lage ist, zu wissen, wo sich die Katheterspitze befindet. Die Transducer sind auf der Sonde gruppenweise angeordnet. Elektronisch geschaltet strahlt jeweils nur eine Gruppe die Ultraschallsignale ab und zwar jeweils die Gruppe, die vom kontinuierlich drehenden Träger aus zum Herz gerichtet ist. Um die benötigte Auflösung bei der Bilderzeugung zu erhalten, sind drei Gruppen zu je acht Transducern vorgesehen. Es besteht das Problem, dass eine starre Sonde, die aufgrund der grossen Anzahl der Transducern relativ lang ist, vom Patienten nicht geschluckt werden kann.

Aufgabe der Erfindung ist es, eine Ultraschallsonde für ein stereoskopisches Abbildungssystem zu schaffen, welche dem Patienten in die Speiseröhre eingeführt werden kann. Diese Aufgabe wird durch die im Anspruch 1 definierte Ultraschallsonde gelöst.

Die Ultraschallsonde ist für ein stereoskopisches Abbildungssystem vorgesehen, das auf einer rasterartigen Aufnahme von Daten und deren elektronischen Verarbeitung beruht. Die Sonde umfasst Gruppen von Schallwandlern, die innerhalb einer elastomeren Hülle auf einem zylinderischen, um eine Longitudinalachse rotierbaren Träger angeordnet sind. Der Träger ist aus mindestens zwei Segmenten zusammengesetzt. Die Segmente sind um einen Kippwinkel gegeneinander kippbar, und zwar um eine Kippachse, die transversal zur Longitudinalachse und vorzugsweise exzentrisch zu dieser liegt. Es gibt einen stabilen Lagezustand, in dem die Segmente unter Einwirkung einer dynamischen und/oder statischen Rückstellkraft eine Lage mit Ausrichtung in Richtung der Longitudinalachse einnehmen.

Die abhängigen Ansprüche 2 bis 9 betreffen vorteilhafte Ausführungsformen der erfindungsgemässen Sonde. Eine Verwendung der erfindungsgemässen Sonde ist Gegenstand des Anspruchs 10.

Nachfolgend wird die Erfindung anhand der Zeichnungen erläutert. Es zeigen:
- Fig. 1: eine erfindungsgemässe Sonde, deren Segmente gegeneinander gekippt sind, - mit Blick senkrecht zu den Kippachsen,
- Fig. 2: die Sonde der Fig. 1 - ohne Schutzhülle - von einem anderen Blickpunkt aus,
- Fig. 3: eine Abwicklung der Sondenoberfläche in eine Ebene,
- Fig. 4: eine Abwicklung für eine zweite Sonde,
- Fig. 5: ein Segmentende mit einer Hälfte eines Scharniergelenks,
- Fig. 6: ein Segmentende, das das Gegenstück zum Segmentende der Fig. 5 ist,
- Fig. 7: eine Seitenansicht des Scharniergelenks der Figuren 5 und 6,
- Fig. 8: eine erste Alternative zum Scharniergelenk und
- Fig. 9: eine zweite Alternative.

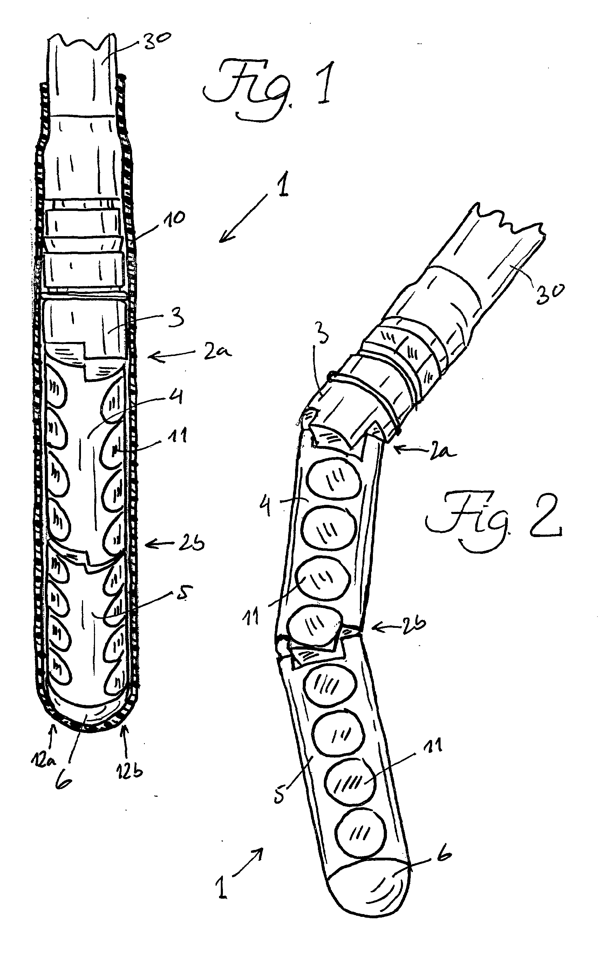

Fig. 1 stellt eine erfindungsgemässe Ultraschallsonde 1 für ein stereoskopisches Abbildungssystem dar, das auf einer rasterartigen Aufnahme von Daten und deren elektronischen Verarbeitung beruht. Die Sonde 1, deren Durchmesser kleiner als 14 mm sein muss, umfasst Schallwandler 11 (Transducer), die Mitglieder von Gruppen 12 sind. Die gezeigte Ausführungsform der Sonde 1, deren Schallwandler 11 jeweils einen Durchmesser von ungefähr 8 mm haben, umfasst drei Gruppen 12a, 12b und 12c mit je acht Schallwandlern 11; die Gruppe 12c befindet sich auf der nicht sichtbaren Seite. Die Schallwandler 11 sind innerhalb einer elastomeren Hülle 10 auf einem zylinderischen, um eine Longitudinalachse rotierbaren Träger angeordnet. Die Hülle 10 ist ein als Schnittbild dargestellter Schutzschlauch, der mit dem Träger der Schallwandler 11 nicht mitdreht. Ein Öl bildet einen Schmierfilm zwischen dem Träger und der Hülle 10. Der Träger ist aus zwei Segmenten 4 und 5 zusammengesetzt. Ein weiteres Segment 3, das auch mitrotiert, enthält ein für die Drehbarkeit des Trägers benötigtes Lager. In einer Spitze 6, die nicht mitrotiert, ist ein zweites Lager angeordnet. Die Segmente 3, 4 und 5 sind mittels Gelenken 2a und 2b jeweils um einen Kippwinkel gegeneinander kippbar.

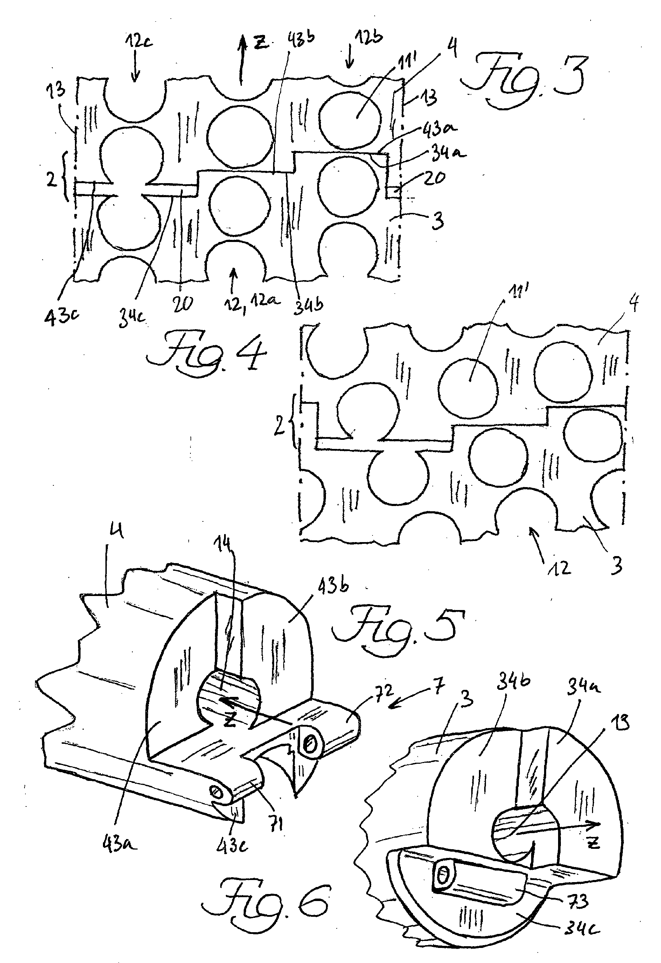

Kippachsen der Gelenke 2a, 2b liegen transversal zur Longitudinalachse und im dargestellten Beispiel exzentrisch zu dieser. Die Longitudinalachse ist eine z-Achse (vgl. Figuren 3, 5 und 6), die sich von einem Sondenanschluss 30 zu der Sondenspitze 6 erstreckt, wenn die Segmente 3, 4 und 5 geradlinig ausgerichtet sind. In Fig. 1, in der die erfindungsgemässe Sonde 1 mit Blick senkrecht zu den Kippachsen gezeigt ist, sind die Segmente 3, 4 und 5 gegeneinander gekippt. Es gibt einen stabilen Lagezustand, in dem die Segmente 3, 4 und 5 unter der Einwirkung einer Rückstellkraft eine Lage mit Ausrichtung in Richtung der Longitudinalachse einnehmen.

Im Beispiel der Fig. 1 kann die elastomere Hülle 10 die Rückstellkraft ausüben. Es ist aber auch möglich, dass ein dehnbares Kabel, das in einem Längskanal der Segmente eingelegt ist, die Rückstellkraft erzeugt.

Die erfindungsgemässe Sonde 1 kann zwei oder mehr Segmente umfassen, auf denen Schallwandler 11 angeordnet sind. Jedes Segment trägt über den ganzen Umfang verteilt eine Anzahl von Schallwandlern 11, die gleich der Anzahl der Gruppen 12 oder ein Mehrfaches dieser Anzahl ist.

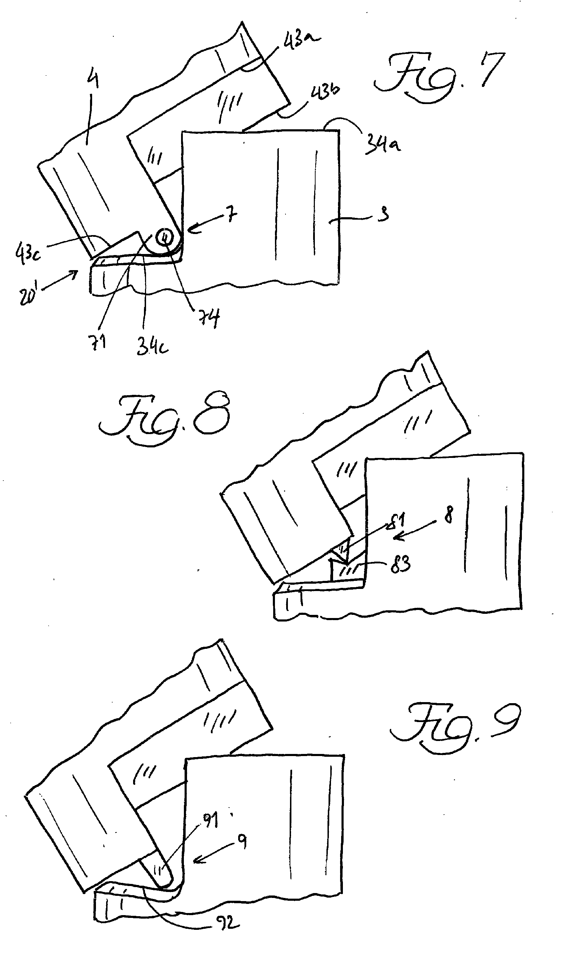

Fig. 3 zeigt als Ausschnitt eine Abwicklung der Sondenoberfläche, welche im stabilen Lagezustand bei einer longitudinalen Linie 13 beginnt bzw. endet und welche in eine Ebene ausgebreitet ist. Die Linie 13 erscheint in der Abwicklung als die beiden strichpunktiert gezeichneten Seitenlinien, die parallel zur z-Achse sind. Die Positionen der Schallwandler 11 sind durch Kreise 11' angegeben. Die Gruppen 12, d.h. die drei Gruppen 12a, 12b und 12c, erstrecken sich in z-Richtung, d.h. in Richtung der Longitudinalachse. Die Schallwandler 11 benachbarter Gruppen 12 sind in z-Richtung jeweils um eine Distanz versetzt, die ungefähr einen Drittel eines Wandlerdurchmesser beträgt. Dank dieser Versetzung ergibt sich eine bessere Auflösung des zu erzeugenden Bildes. Aber aufgrund der Versetzung muss für das Gelenk 2 zwischen den Segmenten 3 und 4 eine etwas aufwändige Form gewählt werden. Die räumliche Ausbildung dieser Form ist in den Figuren 5 und 6 illustriert:

Fig. 5 zeigt ein Ende des Segments 4 mit einer Hälfte einer scharnierartigen Verbindung 7. Fig. 6 zeigt ein Ende des Segments 3, welches das Gegenstück zum Segment 4 mit der zweiten Hälfte des Scharniergelenks 7 ist. Fig. 7 zeigt als Seitenansicht das zusammengesetzte Scharniergelenk 7 in Kippstellung. Ein in zwei Ösen 71 und 72 des Segments 4 gelagerter Verbindungsstift 74 bildet die Kippachse. Der Verbindungsstift 74 ist in einer Halterung 73 des Segments 3 befestigt. Zwischen den benachbarten Segmenten 3 und 4 werden durch die exzentrisch angeordnete Kippachse zwei verschieden grosse Bereiche voneinander abgegrenzt. Nach dem Zusammensetzen des Scharniergelenks 7 bilden die beiden Segmente 3 und 4 im grösseren dieser Bereiche zumindest angenähert einen Formschluss, wenn die Segmente 3 und 4 sich im stabilen Lagezustand befinden. Bei diesem Formschluss kommen Teilflächen 43a und 43b auf entsprechende Teilflächen 34a bzw. 34b zu liegen. Die Kontaktstellen dieser Teilflächen sind in der Abwicklung der Fig. 3 als treppenförmige Linie zu erkennen. Zwischen weiteren Teilflächen 43c und 34c bleibt eine Lücke 20 bestehen. Durch die Breite dieses Lücke 20 ist der maximale Kippwinkel gegeben. Dieser kann bis zu 30° betragen. Die Lage der Lücke 20 ist in Fig. 7 mit dem Bezugszeichen 20' angegeben.

In Fig. 4 ist eine Abwicklung für eine zweite Sonde 1, bei der die Gruppen 12 nicht longitudinal ausgerichtet sind, sondern die Schallwandler 11 auf helixförmigen Kurven angeordnet sind.

Fig. 8 zeigt eine erste Alternative 8 zum Scharniergelenk 7: Die Kippachse wird durch eine Schneide 81 gebildet, die in einer Kerbe 83 gelagert ist. Die Schneide 81 lässt sich auf der Kerbe 83 so bewegen, dass die benötigten Kippstellungen herstellbar sind. Damit die benachbarten Segmente 3 und 4 miteinander verbunden bleiben, muss ein elastisches Kabel vorgesehen sein (nicht dargestellt). Das Kabel ist an einem ersten Ende in der Spitze 6 befestigt und führt beispielsweise durch zentrale Kanäle 14, 13 (siehe Fig. 5, 6) zum Segment 3, wo das zweite Ende des Kabels befestigt ist. Das Kabel kann auch in einem zusätzlichen, nicht dargestellt Kanal eingelegt sein. Die zentralen Kanäle 14, 13 sind an sich für elektrische Verbindungsleitungen zu den Schallwandlern 11 vorgesehen.

Eine zweite Alternative 9 ist in Fig. 9 skizziert. Auch hier wird ein elastisches Kabel benötigt. Die Kippachse ist durch die Berührungslinie zwischen einem Steg 91, der eine runde Kante aufweist, und einer ebenen Lagerfläche 92 gegeben. Beim Kippen rollt der Steg 91 mit seiner runden Kante auf der Lagerfläche 92 ab. Dabei bewegt sich offensichtlich die Kippachse. Die Lagerfläche 92 kann gegenüber der Longitudinalachse einen Winkel einschliessen, der etwas kleiner als 90° ist. In diesem Fall macht die Kippachse eine zusätzliche Bewegung in Richtung der Longitudinalachse, wodurch sich die Rückstellkraft des Kabels verstärkt und somit die Stabilität des stabilen Lagezustands der Segmente 3 und 4 erhöht wird.

Eine zwischen den Segmenten wirkende Rückstellkraft muss relativ klein sein, so dass die Sonde ohne grosse Widerstände in die Speiseröhre eingeführt werden kann. Eine solche Rückstellkraft ist in der Regel zu gering, um in der Messposition den stabilen Lagezustand der Segmente 3 und 4 exakt herzustellen. Ist die Abweichung von einer geradlinigen Ausrichtung nicht zu gross, so lässt sich der mit den Segmenten 3 und 4 gebildete Träger drehen. Sobald der Träger gedreht wird, bildet sich dynamisch eine zusätzliche Rückstellkraft aus, die den Träger in den geradlinigen Lagezustand zwingt. Durch die Drehung des Trägers (15 bis 20 Umdrehungen pro Sekunde) wird also dieser Lagezustand vollständig stabilisiert. Es liegt somit ein stabiler Lagezustand vor, in dem die Segmente unter Einwirkung einer dynamischen und einer statischen Rückstellkraft eine Lage mit Ausrichtung in Richtung der Longitudinalachse einnehmen. Der Lagezustand kann auch lediglich durch eine dynamische oder durch eine statische Rückstellkraft stabilisiert werden.

Der Schallwandler 11 hat einen Durchmesser im Bereich von 6 bis 10 mm. Dieser Durchmesser ist vorzugsweise ungefähr 8 mm. Das Verhältnis zwischen den Durchmessern des Trägers und des Schallwandlers liegt im Bereich von 1.2 bis 1.8; vorzugsweise beträgt dieses Verhältnis ungefähr 1.5.

## Patentansprüche

1. Ultraschallsonde (1) für ein stereoskopisches Abbildungssystem, das auf einer rasterartigen Aufnahme von Daten und deren elektronischen Verarbeitung beruht, welche Sonde Gruppen (12) von Schallwandlern (11) umfasst, die innerhalb einer elastomeren Hülle (10) auf einem zylinderischen, um eine Longitudinalachse rotierbaren Träger angeordnet sind, **dadurch gekennzeichnet, dass** der Träger aus mindestens zwei Segmenten (3, 4) zusammengesetzt ist, dass die Segmente um einen Kippwinkel gegeneinander kippbar sind, und zwar um eine Kippachse, die transversal zur Longitudinalachse und vorzugsweise exzentrisch zu dieser liegt, und dass es einen stabilen Lagezustand gibt, in dem die Segmente unter Einwirkung einer dynamischen und/oder statischen Rückstellkraft eine Lage mit Ausrichtung in Richtung der Longitudinalachse einnehmen.

2. Sonde nach Anspruch 1, **dadurch gekennzeichnet, dass** die elastomere Hülle (10) eine Rückstellkraft ausübt.

3. Sonde nach Anspruch 1 oder 2, **dadurch gekennzeichnet, dass** ein dehnbares Kabel, das in einem Längskanal (13, 14) der Segmente (3, 4) eingelegt ist, eine Rückstellkraft ausübt.

4. Sonde nach einem der Ansprüche 1 bis 3, **dadurch gekennzeichnet, dass** die Segmente (3, 4) starr sind, eine scharnierartige Verbindung (7) zwischen zwei benachbarten Segmenten besteht und ein in zwei Ösen (71, 72) gelagerter Verbindungsstift (74) der scharnierartigen Verbindung die Kippachse bildet.

5. Sonde nach einem der Ansprüche 1 bis 4, **dadurch gekennzeichnet, dass** zwei oder mehr Segmente (3, 4, 5) vorliegen und dass jedes Segment über den ganzen Umfang verteilt eine Anzahl von Schallwandlern (11) trägt, die gleich der Anzahl der Gruppen (12) oder ein Mehrfaches dieser Anzahl ist.

6. Sonde nach einem der Ansprüche 1 bis 5, **dadurch gekennzeichnet, dass** mehr als zwei gegeneinander kippbare Segmente (3, 4, 5) vorliegen und dass die Kippachsen parallel zueinander ausgerichtet sind.

7. Sonde nach einem der Ansprüche 1 bis 6, **dadurch gekennzeichnet, dass** der maximale Kippwinkel einen Wert bis zu 30° hat.

8. Sonde nach einem der Ansprüche 1 bis 7, **dadurch gekennzeichnet, dass** zwischen benachbarten Segmenten (3, 4) durch die exzentrisch angeordnete Kippachse zwei verschieden grosse Bereiche voneinander abgegrenzt sind und dass die beiden Segmente im grösseren dieser Bereiche zumindest angenähert einen Formschluss bilden, wenn sie sich im stabilen Lagezustand befinden.

9. Sonde nach einem der Ansprüche 1 bis 8, **dadurch gekennzeichnet, dass** auf dem Träger drei Gruppen (12a, 12b, 12c) von Schallwandlern (11) jeweils in einer festen oder variablen Richtung angeordnet sind, die parallel zur Longitudinalachse bzw. geneigt zu dieser ist, dass jeder Schallwandler einen Durchmesser im Bereich von 6 bis 10 mm hat, dieser Durchmesser vorzugsweise ungefähr 8 mm ist, und dass das Verhältnis zwischen den Durchmessern des Trägers und des Schallwandlers im Bereich von 1.2 bis 1.8, vorzugsweise bei ungefähr 1.5 ist.

10. Verwendung einer Sonde gemäss einem der Ansprüche 1 bis 9, **dadurch gekennzeichnet, dass** an einem menschlichen oder tierischen Körper aus einem Körperinnenraum heraus dreidimensionale Darstellungen von einem sich bewegenden Organ, insbesondere vom Herzen, mit einem elektronischen Abbildungssystem und in Echtzeit hergestellt werden, wobei der Körperinnenraum von aussen zugänglich ist, der Körperinnenraum insbesondere die Speiseröhre ist.
